# EUROPÄISCHE PATENTSCHRIFT

(11) **EP 2 249 735 B1**
(45) Veröffentlichungstag und Bekanntmachung des Hinweises auf die Patenterteilung: **18.03.2015**
(21) Anmeldenummer: 09703052.2
(22) Anmeldetag: 09.01.2009
(51) Int. Cl.: A61B 18/12

(54) **ELEKTROCHIRURGIEGERÄT**
ELECTRO-SURGICAL DEVICE
APPAREIL ÉLECTROCHIRURGICAL

(30) Priorität: 14.01.2008 DE 102008004241
(43) Veröffentlichungstag der Anmeldung: 17.11.2010
(60) Teilanmeldung: 14190053.0
(73) Patentinhaber: ERBE Elektromedizin GmbH, 72072 Tübingen (DE)
(72) Erfinder: SELIG, Peter, 72379 Hechingen (DE)
(74) Vertreter: Bohnenberger, Johannes
(86) Internationale Anmeldenummer: PCT/EP2009/000088
(87) Internationale Veröffentlichungsnummer: WO 2009/090017

(56) Entgegenhaltungen:
- WO-A-2008/015957
- DE-A1- 4 135 180
- DE-A1- 10 100 385
- FR-A- 2 824 480
- FR-A- 2 829 398
- GB-A- 2 408 210
- US-A- 4 154 240
- US-A- 4 473 075
- US-A1- 2002 022 836
- US-A1- 2004 267 333
- US-A1- 2007 032 827

## Beschreibung

Die Erfindung betrifft ein Elektrochirurgiegerät nach dem Oberbegriff des Patentanspruches 1.

Ein Elektrochirurgiegerät gemäß dem Oberbegriff des Anspruchs 7 ist aus der US 4 473 075 bekannt. In der modernen Chirurgie werden häufig derartige Elektrochirurgiegeräte verwendet, die einen Hochfrequenzgenerator zur Erzeugung eines hochfrequenten Wechselstroms beinhalten. Dieser hochfrequente Wechselstrom wird dann verwendet, um biologisches Gewebe zu schneiden, zu koagulieren oder in anderer Form zu behandeln.

Die Grundfrequenz des HF-Generators liegt üblicherweise zwischen 300 kHz und 4 MHz. Dieser hochfrequente Strom wird je nach Anwendung in verschiedenen Formen hinsichtlich seiner Amplituden moduliert. Üblicherweise erfolgt eine Pulsdauermodulation, um die applizierte Leistung zu bestimmen. Hierbei wird das Verhältnis von burstförmigen Signalabschnitten zu nachfolgenden Pausen eingestellt. Die Taktfrequenz bzw. Modulation erfolgt üblicherweise mit Frequenzen zwischen 1 kHz und 50 kHz.

Weiterhin wird zu einer Leistungsdosierung, welche es dem Operateur ermöglicht, den Behandlungsfortschritt zu verfolgen, eine weitere Modulation (An- und Abschalten des Ausgangssignals) mit sehr niedrigen Frequenzen zwischen 1 Hz und 10 Hz durchgeführt.

Schließlich wird zur Vermeidung von Leckströmen bzw. Verlusten des Generators während des Leerlaufes bei fehlender Last mit sehr niederfrequenten Pulsen, also Schwingungspaketen der Grundfrequenz gearbeitet.

Neben den elektrochirurgischen Geräten befindet sich nun im Operationssaal eine Vielzahl von anderen elektrischen bzw. elektronischen Geräten im Betrieb. Dies sind insbesondere Patientenüberwachungsgeräte (z.B. EEG-Geräte) oder Videogeräte. Ein typisches Beispiel für ein Videogerät, das für eine Operation unabdingbar notwendig ist, findet sich bei endoskopisch durchgeführten Operationen. In diesem Fall liegt der Videochip in unmittelbarer Nähe des Ortes, an welchem mittels eines elektrochirurgischen Instrumentes der oben beschriebene hochfrequente Wechselstrom am Gewebe appliziert wird.

In allen oben genannten Fällen kommt es häufig zu Störungen der elektronischen Geräte, die bis hin zum Ausfall des verwendeten Patientenmonitors führen können, so dass über eine gewisse Zeit hin keine Überwachung der Vitalparameter des Patienten möglich ist. Gleiches gilt für das Videobild. In allen Fällen können derartige Probleme fatale Folgen für den Patienten haben.

Es ist Aufgabe der vorliegenden Erfindung, ein Verfahren bzw. ein Elektrochirurgiegerät der eingangs genannten Art dahin gehend aufzuzeigen, dass Störungen insbesondere peripherer Geräte vermieden oder zumindest verringert werden.

Diese Aufgabe wird durch ein Elektrochirurgiegerät nach Anspruch 1 gelöst.

Vorrichtungsmäßig wird die oben genannte Aufgabe durch ein Elektrochirurgiegerät mit einem Hochfrequenzgenerator gelöst, der ein hochfrequentes Ausgangssignal zur Behandlung, insbesondere zum Schneiden oder Koagulieren von biologischem Gewebe erzeugt, wobei der Hochfrequenzgenerator so ausgebildet ist, dass das Ausgangssignal eine vorbestimmte Signalfrequenz aufweist und kontinuierlich, mit einer Modulationsfrequenz moduliert oder in Bursts mit vorbestimmtem Taktverhältnis von Signal zu Pause und einer vorbestimmten Taktfrequenz erzeugt wird. Hierbei ist ein Modulationsgenerator vorgesehen, der ein niederfrequentes Modulationssignal erzeugt, welches dem HF-Generator zur Steuerung des Ausgangssignals so zugeführt wird, dass die Signalfrequenz und/oder die Modulationsfrequenz und/oder die Taktfrequenz mit dem niederfrequenten Modulationssignal moduliert werden, so dass deren Spektren verbreitert werden.

Ein wesentlicher Punkt der Erfindung liegt also darin, dass die bisher konstant gehaltene Modulationsfrequenz bzw. Taktfrequenz, aber auch die Signalfrequenz, gemäß der vorliegenden Erfindung nicht bei einem konstanten Wert gehalten werden. Dadurch kommt es bei einer Betrachtung im Frequenzbereich zu einer Verbreiterung der (durch konstante Frequenzen erzeugten) Spektrallinien zu Spektralbändern. Diese "Frequenzspreizung" führt zu erheblich verminderten Spitzenwerten der Leistung, da die erzeugte Energie eben nicht bei einer einzigen Frequenz, sondern verteilt auf eine Vielzahl von Frequenzen bzw. ein Frequenzband erzeugt wird. Dadurch wiederum werden sowohl leitungsgebundene als auch drahtlose Störungsaussendungen erheblich reduziert, was die Einhaltung der wichtigen EMV-Grenzwerte des Gerätes günstig beeinflusst. Damit wiederum erfolgt eine direkte Reduzierung der elektrischen Störungen von Patientenmonitoren, Videosystemen und anderen elektrischen bzw. elektronischen Geräten mit all ihren beschriebenen problematischen Folgen. Für die chirurgischen Effekte, die allesamt sehr träge sind, da sie in erster Linie auf thermischen Effekten beruhen, spielt diese zusätzliche Modulation der Signale keinerlei Rolle.

Die vorteilhaften Ausführungsformen der Erfindung wurden anhand des eingangs beschriebenen Verfahrens bereits erläutert und gelten natürlich auch für das hier beanspruchte Elektrochirurgiegerät.

Nachfolgend wird ein Ausführungsbeispiel der Erfindung anhand von Abbildungen näher erläutert.

Hierbei zeigen
- Fig. 1 ein Blockschaltbild einer Ausführungsform des erfindungsgemäßen Elektrochirurgiegerätes und
- Fig. 2-4 verschiedene Signalformen mit den dazu gehörigen Spektren.

In der nachfolgenden Beschreibung werden für gleiche und gleich wirkende Teile dieselben Bezugsziffern verwendet.

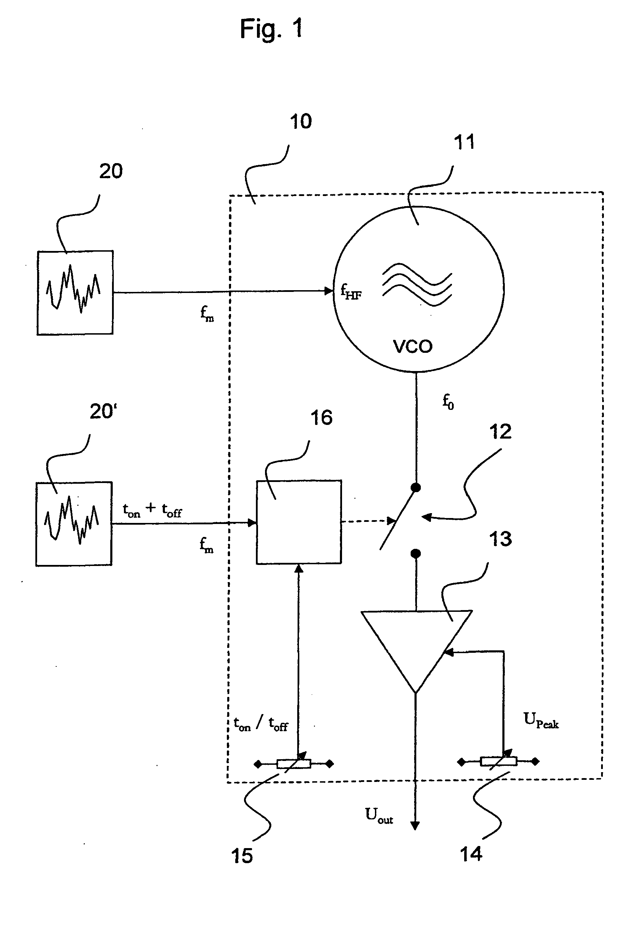

In Fig. 1 ist eine Ausführungsform eines Elektrochirurgiegerätes gemäß der vorliegenden Erfindung stark schematisiert gezeigt. Details derartiger bekannter Elektrochirurgiegeräte finden sich beispielsweise in der DE 199 43 792 C2, DE 100 46 592 C2, EP 0 430 929 B1 oder EP 0 653 192 B1, auf die hier ausdrücklich Bezug genommen wird.

Ein solches Elektrochirurgiegerät weist einen Hochfrequenzgenerator 10 auf, der einen Oszillator 11 umfasst, dessen Ausgangssignal mit der Frequenz f₀ über einen Schalter 12 geschaltet und durch einen Ausgangsverstärker 13 verstärkt wird, so dass am Ausgang des HF-Generators 10 ein Ausgangssignal Uₒᵤₜ ansteht. Es sei hier betont, dass dies eine schematische Darstellung zur Erläuterung der Funktionsweise der Anordnung ist.

Zur Einstellung der Ausgangsamplitude Uₚₑₐₖ ist ein Einstellorgan 14 vorgesehen, welches den Verstärkungsfaktor des Ausgangsverstärkers 13 einstellt. Zur Steuerung des Schalters 12 ist eine Schaltersteuerung 16 vorgesehen, die über ein Einstellorgan 15 das Tastverhältnis einstellt, also die Zeitdauer tₒₙ, während derer das Ausgangssignal des Oszillators 11 am Eingang des Ausgangsverstärkers 13 anliegt, dividiert durch die Zeit t_{off}, während derer der Schalter 12 geöffnet ist.

Der Oszillator 11 ist im vorliegenden Beispiel als VCO dargestellt, also als Oszillator, dessen Schwingungsfrequenz f_{HF} durch eine Spannung eingestellt werden kann. Diese Spannung wird von einem Modulationsgenerator 20 vorgegeben.

Die Schaltersteuerung 16 weist einen zweiten Eingang auf, über welchen ein Signal tₒₙ + t_{off} aufgenommen und so verarbeitet wird, dass der Schalter 12 mit dem vorgegebenen Tastverhältnis tₒₙ / t_{off} der Periode tₒₙ + t_{off} gesteuert wird. Dieses "Periodensignal" stellt ein Modulationssignal dar, welches von einem Modulationsgenerator 20' erzeugt wird.

Nachfolgend wird die Funktionsweise dieser schematisiert dargestellten Ausführungsform der Erfindung erläutert.

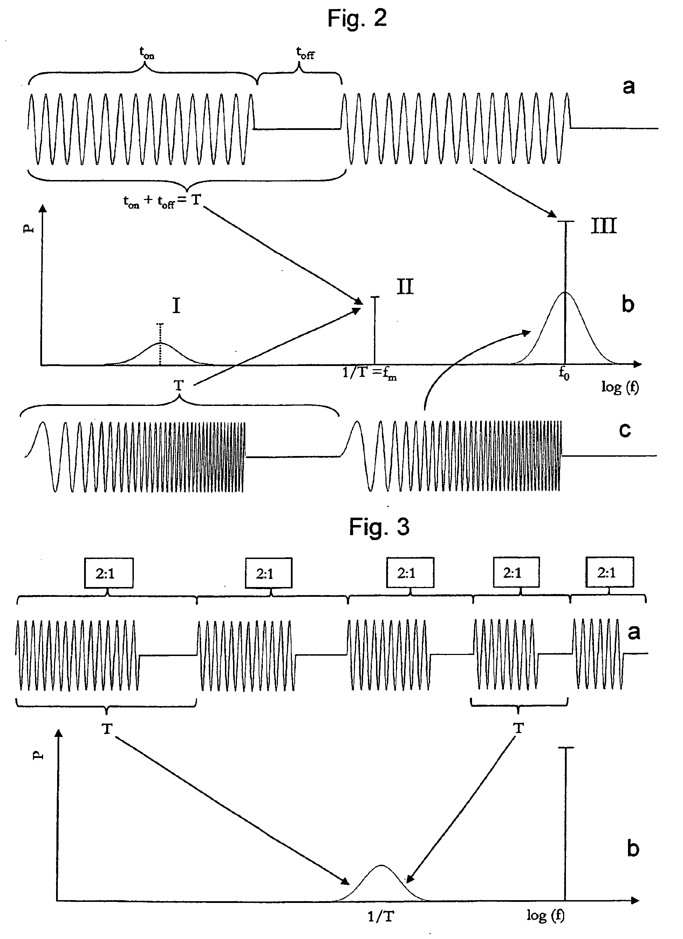

Fig. 2 ist der zeitliche Verlauf eines durch den Schalter 12 mit einer Periode T=tₒₙ + t_{off} modulierten Hochfrequenzsignals mit der Frequenz f₀ gezeigt. Bei der in Fig. 2b gezeigten Darstellung handelt es sich um ein Frequenzspektrum, bei welchem die Leistung P des Signals über die Frequenz zu sehen ist. Das in Fig. 2a gezeigte konventionell modulierte Hochfrequenzsignal führt einerseits zu einer scharfen Spektrallinie, die in Fig. 2b mit III bezeichnet ist. Die Modulationsperiode T führt zu einer Spektrallinie, die in Fig. 2b mit II bezeichnet ist.

Weiterhin ist in Fig. 2b noch eine Spektrallinie I gezeigt, die im sehr niederfrequenten Bereich liegt und aus der eingangs beschriebenen Signalmodulation herrührt, die während "inaktiver" Perioden der Benutzung vorgenommen wird, um die Verluste gering zu halten.

In Fig. 2c ist ein Signal gezeigt, das gemäß der vorliegenden Erfindung einerseits eine konstante Modulationsperiode T aufweist, bei dem aber andererseits das Hochfrequenzsignal nicht mit einer konstanten Frequenz f₀ vorliegt, sondern gewobbelt wird. Die Frequenz steigt also über die Zeit an und beginnt bei jeder neuen Periode wieder mit der niedrigeren Frequenz. Dieses gewobbelte Signal gemäß Fig. 2c führt zu dem in Fig. 2b gezeigten Spektrum rings um die Spektrallinie III, da niedrigere Frequenzanteile und höhere Frequenzanteile vorliegen. Nachdem aber die Modulationsfrequenz für das An- und Ausschalten des Signals gegenüber Fig. 2a unverändert ist, bleibt die Spektrallinie II in Fig. 2b bestehen.

In Fig. 3a ist ein Signal gezeigt, bei welchem das Hochfrequenzsignal in seiner Frequenz f₀ unverändert ist. Weiterhin liegt bei dem Signal nach Fig. 3a ein konstantes Tastverhältnis tₒₙ/t_{off}=2/1 vor, wobei die Periodendauer T der Einzelsignale bestehend aus einem Burst und einer nachfolgenden Pause zeitlich variieren. Dies führt zu einem Spektrum, wie dies in Fig. 3b gezeigt ist, bei welchem die niedrigere Frequenz, resultierend aus der Periodendauer T als Spektrum erscheint, das hochfrequente Signal jedoch eine scharfe Spektrallinie erzeugt.

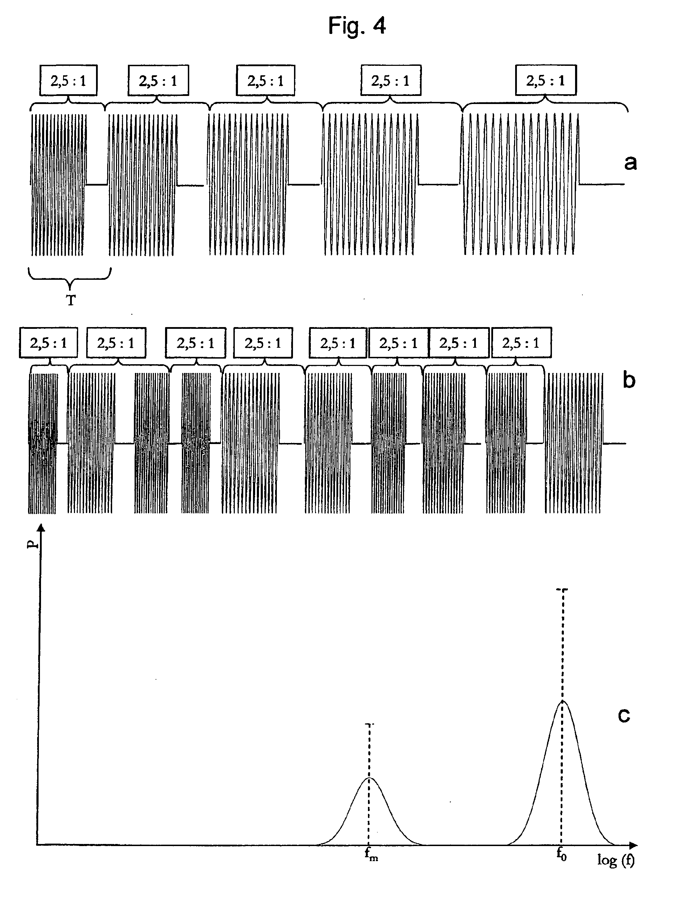

In Fig. 4a sind nun Bursts gezeigt, bei denen das Tastverhältnis immer tₒₙ/t_{off}=2,5/1 beträgt. Die Hochfrequenzsignale innerhalb dieser Bursts weisen eine variierende Frequenz auf. Weiterhin sind die Periodendauern T unterschiedlich.

Der Signalverlauf nach Fig. 4b unterscheidet sich nach dem von Fig. 4a dadurch, dass die Periodendauern T nicht kontinuierlich ansteigen sondern zufällig variieren. Ansonsten beträgt auch beim Signal nach Fig. 4b das Tastverhältnis immer 2,5/1.

Diese beiden, in den Fig. 4a und 4b gezeigten Signale führen nun zu einem Spektrum, wie es in Fig. 4c gezeigt ist. Hieraus ist zu ersehen, dass die scharfen Spektrallinien, welche dem Hochfrequenzsignal bzw. der Modulations-Taktfrequenz entsprechen, verschwunden sind und durch breite Spektren ersetzt werden.

Vergleicht man nun das aus einem konventionellen elektrochirurgischen Generator stammende Signal gemäß Fig. 2a und das daraus resultierende Spektrum gemäß Fig. 2b bzw. 3c, so wird ersichtlich, dass die Maximalleistungen von scharfen Spektrallinien, wie sie aus kontinuierlichen Hochfrequenzsignalen bzw. einer Modulation mit einer konstanten Frequenz erfolgen, bei Variation der Signalfrequenz bzw. Modulationsfrequenz erheblich absinken. Dadurch wird das angestrebte Ziel erreicht, die Störstrahlung bzw. die netzgebundenen Störanteile, die auf periphere Geräte nachteilige Einflüsse haben könnten, erheblich abzusenken. Hierbei genügt es, wenn die Frequenz des HF-Signals bzw. die Modulationsfrequenz nur um wenige Prozent um ihren Mittelwert schwanken, um die beschriebene Frequenzspreizung, also die Aufweitung einzelner Spektrallinien, in ein verbreitetes Spektrum zu erzielen.

Diese Steuerung der Signal- bzw. Modulationsfrequenz kann durch die Modulationsgeneratoren 20 und 20' entweder regelmäßig, z.B. mit einem sinusförmigen Signal oder mit einem beliebigen anderen, insbesondere mit einem Rauschsignal erfolgen. Erfolgt die Modulation mit einem sinusförmigen Signal, so ergibt sich im Ausgangsspektrum wieder eine neue Spektrallinie (in den Abbildungen nicht gezeigt). Wenn diese Spektrallinie so gelegt wird, dass sie innerhalb eines, periphere Geräte wenig störenden Bereiches liegt, z.B. bei der Netzfrequenz, so ist nur eine geringe Störung der peripheren Geräte zu erwarten, da diese üblicherweise in eben diesem Frequenzbereich ohnehin schon entstört sind. Wenn die Modulationsgeneratoren 20, 20' stochastisch modulieren, so wird wiederum ein "Störspektrum" erzeugt, das jedoch in seiner Amplitude aufgrund der soeben beschriebenen Verbreiterung sehr gering ist. Auch hier kann die Mittenfrequenz des Rauschsignals vorteilhafterweise in einen Bereich gelegt werden, in welchem die vor Störung zu schützenden peripheren Geräte "unempfindlich" sind.

Aus Obigem geht hervor, dass ein wesentliches Prinzip der Erfindung darin liegt, dass periodische Vorgänge, also das eigentliche Hochfrequenzsignal und sämtliche Modulationsvorgänge, die zur Erzeugung besonderer Effekte angewendet werden, nicht konstant gehalten sondern zeitlich variiert werden, um scharfe Spektrallinien mit hohen Leistungen zu vermeiden.

### Bezugszeichenliste

- 10: HF-Generator
- 11: Oszillator
- 12: Schalter
- 13: Ausgangsverstärker
- 14: Amplitudeneinstellorgan
- 15: Tastverhältniseinstellorgan
- 16: Schaltersteuerung
- 20, 20': Modulationsgenerator

## Patentansprüche

1. Elektrochirurgiegerät mit einem Hochfrequenzgenerator, der ein hochfrequentes Ausgangssignal (Uₒᵤₜ; f₀) zur Behandlung, insbesondere zum Schneiden oder Koagulieren von biologischem Gewebe erzeugt, wobei der Hochfrequenzgenerator (10) so ausgebildet ist, dass das Ausgangssignal (Uₒᵤₜ) eine vorbestimmte Signalfrequenz (f₀) aufweist und kontinuierlich, mit einer Modulationsfrequenz (fₘ) moduliert oder mit Bursts mit vorbestimmtem Tastverhältnis von Signal (tₒₙ) zu Pause (t_{off}) und einer vorbestimmten Taktfrequenz (fₘ) erzeugt wird,
**dadurch gekennzeichnet, dass**
ein Modulationsgenerator (20, 20') vorgesehen ist, der ein niederfrequentes Modulationssignal erzeugt, welches den HF-Generator (10) zur Steuerung des Ausgangssignals (Uₒᵤₜ; f₀) so zugeführt wird, dass die Signalfrequenz und/oder die Modulationsfrequenz und/oder die Taktfrequenz mit dem niederfrequenten Modulationssignal moduliert werden, so dass deren Spektren verbreitert werden.

2. Elektrochirurgiegerät nach Anspruch 1,
**dadurch gekennzeichnet, dass**
das Modulationssignal in einem Frequenzbereich außerhalb von solchen Frequenzen erzeugt wird, die bei peripheren medizinischen Geräten, insbesondere Patientenüberwachungssystemen oder Videosystemen Störungen verursachen.

3. Elektrochirurgiegerät nach einem der Ansprüche 1 oder 2,
**dadurch gekennzeichnet, dass**
das Modulationssignal einen im Wesentlichen stetigen Verlauf aufweist.

4. Elektrochirurgiegerät nach einem der Ansprüche 1-3,
**dadurch gekennzeichnet, dass**
das Modulationssignal ein Zufalls- oder Rauschsignal ist.

5. Elektrochirurgiegerät nach einem der Ansprüche 1-4,
**dadurch gekennzeichnet, dass**
das Modulationssignal ein vorzugsweise stetig wobbelndes Signal ist.

6. Elektrochirurgiegerät nach einem der Ansprüche 1-5,
**dadurch gekennzeichnet, dass**
das Modulationssignal derart ausgebildet ist, dass die Signalfrequenz und/oder die Modulationsfrequenz und/oder die Taktfrequenz um weniger als 20 %, vorzugsweise weniger als 10 %, vorzugsweise weniger als 5 % variiert werden.

## Claims

1. Electrosurgical device, comprising a radiofrequency generator, which generates a radiofrequency output signal (Uₒᵤₜ; f₀) for treating, in particular for cutting or coagulating, biological tissue, wherein the radiofrequency generator (10) is designed in such a way that the output signal (Uₒᵤₜ) has a predetermined signal frequency (f₀) and is generated in a continuous manner, in a manner modulated by a modulation frequency (fₘ) or with bursts having a predetermined pulse/pause ratio of signal (tₒₙ) to pause (t_{off}) and a predetermined clock frequency (fₘ),
**characterized in that**
provision is made for a modulation generator (20, 20'), which generates a low frequency modulation signal, which is fed in such a way to the RF generator (10) for controlling the output signal (Uₒᵤₜ; f₀) that the signal frequency and/or the modulation frequency and/or the clock frequency are modulated by the low frequency modulation signal, such that the spectra thereof are broadened.

2. Electrosurgical device according to Claim 1,
**characterized in that**
the modulation signal is generated in a frequency range away from those frequencies that cause interference in peripheral medical devices, in particular patient monitoring systems or video systems.

3. Electrosurgical device according to either of Claims 1 and 2,
**characterized in that**
the modulation signal has a substantially continuous profile.

4. Electrosurgical device according to one of Claims 1-3,
**characterized in that**
the modulation signal is a random signal or noise signal.

5. Electrosurgical device according to one of Claims 1-4,
**characterized in that**
the modulation signal is a preferably continuously sweeping signal.

6. Electrosurgical device according to one of Claims 1-5,
**characterized in that**
the modulation signal is configured in such a way that the signal frequency and/or the modulation frequency and/or the clock frequency are varied by less than 20%, preferably less than 10%, preferably less than 5%.

## Revendications

1. Appareil électrochirurgical comportant un générateur à haute fréquence qui génère un signal de sortie à haute fréquence (Uₒᵤₜ ; f₀) destiné à effectuer un traitement, notamment à pratiquer une incision ou une coagulation de tissu biologique, dans lequel le générateur à haute fréquence (10) est conçu de telle manière que le signal de sortie (Uₒᵤₜ) présente une fréquence de signal prédéterminée (f₀), soit modulé à une fréquence de modulation (fₘ) ou soit généré avec des salves présentant un rapport cyclique prédéterminé du signal (tₒₙ) à pause (t_{off}) et à une fréquence d'horloge prédéterminée (fₘ),
**caractérisé en ce qu'**il est prévu un générateur de modulation (20, 20') qui génère un signal de modulation à basse fréquence délivré au générateur HF (10) pour commander le signal de sortie (Uₒᵤₜ ; f₀) de telle manière que la fréquence du signal et/ou la fréquence de modulation et/ou la fréquence d'horloge soient modulées avec le signal de modulation à basse fréquence afin que leurs spectres soient élargis.

2. Appareil électrochirurgical selon la revendication 1, **caractérisé en ce que** le signal de modulation est généré dans un domaine de fréquence extérieur à des fréquences qui provoquent des perturbations d'appareils médicaux périphériques, notamment de systèmes de surveillance de patients ou de systèmes vidéo.

3. Appareil électrochirurgical selon les revendications 1 ou 2, **caractérisé en ce que** le signal de modulation présente une évolution sensiblement constante.

4. Appareil électrochirurgical selon l'une quelconque des revendications 1-3, **caractérisé en ce que** le signal de modulation est un signal aléatoire ou de bruit.

5. Appareil électrochirurgical selon l'une quelconque des revendications 1-4, **caractérisé en ce que** le signal de modulation est un signal présentant de préférence une ondulation constante.

6. Appareil électrochirurgical selon l'une quelconque des revendications 1-5, **caractérisé en ce que** le signal de modulation est conçu de telle manière que la fréquence de signal et/ou la fréquence de modulation et/ou la fréquence d'horloge soient amenées à varier de moins de 20%, de préférence, de moins de 10 %, et mieux encore, de moins de 5 %.
